# EUROPEAN PATENT APPLICATION

(11) **EP 2 305 257 A1**
(43) Date of publication of application: **06.04.2011**
(21) Application number: 10011435.4
(22) Date of filing: 08.07.2003
(51) Int. Cl.: A61K 31/44, A61P 35/00

(54) **Amonafide salts and compositions comprising same**

(30) Priority: 08.07.2002 US 394558 P
(62) Divisional of application: 03763417.7
(71) Applicant: ChemGenex Pharmaceuticals, Inc., Menlo Park CA 94025 (US)
(72) Inventor: Brown, Dennis M., Menlo Park CA 94025 (US)
(74) Representative: Bailey, Sam Rogerson

(57) **Abstract**

The present invention concerns novel methods for the synthesis of napthalimides and mitonafide analogs, as well as salts thereof. Also included are novel compositions, including napthalimides and napthalimide salts, analogs thereof, as well as stable liquid dosage forms thereof.

## Description

This application claims the benefit of U.S. Provisional Application Number 60/394,558, filed July 8, 2002.

### FIELD OF THE INVENTION

The present invention provides processes for the synthesis of naphthalimides, including amonafide, amonafide salts, and analogs thereof. The invention also provides compositions comprising amonafide salts or chemical intermediates.

### BACKGROUND OF THE INVENTION

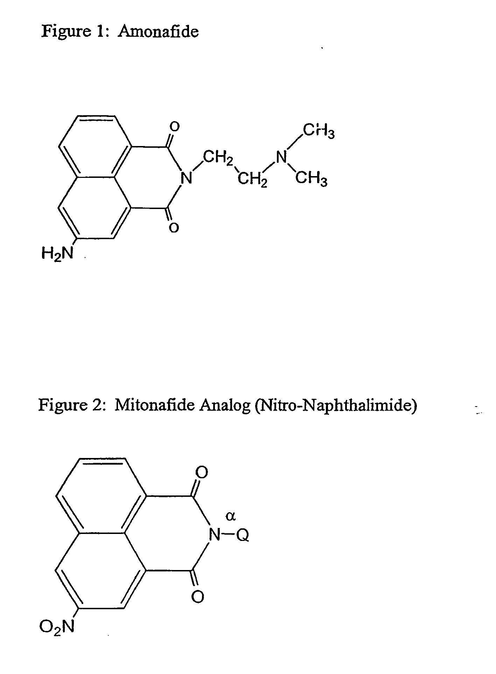

Amonafide, a naphthalimide analog, is a known antitumor compound. U.S. Patent 4,204,063 (hereby expressly incorporated by reference in its entirety) describes that amonafide may be prepared by reaction of 3-amino-1,8-naphthalic anhydride and 2-dimethylaminoethylamine in ethanol. The product is filtered and recrystallized in chloroform-n-hexane. The product exhibits in the form of very fine needle-like yellow crystals with the structure shown in Figure 1.

The starting material (3-amino-1,8-naphthalic anhydride), however, is not readily available in commercial quantities, and requires additional syntheses for small scale and large-scale manufacturing.

Amonafide as a free base is very poorly soluble in water. Consequently, it is difficult to formulate either oral or injectable pharmaceutical dosage forms of amonafide.

Attempts have been reported in the scientific and patent literature to synthesize amonafide salts to improve solubility, and thereby improve the feasibility of formulating suitable dosage forms. As disclosed in US Patent No. 5,420,137 (hereby expressly incorporated by reference in its entirety), monohydrochloride and monomethanesulfonate salts can be prepared by reacting amonafide with a calculated amount of hydrochloric acid or monomethanesulfonic acid in an alcoholic solution. For example, U.S. Patent 5,420,137 indicates that a mol ratio of amonafide and hydrochloric acid is about 1:1.1. However, reaction with a calculated amount of hydrochloric acid to formulate amonafide monohydrochloride salt is troublesome and difficult to control. Additionally, Rosenberg, J. et al. (German Patent No. 4023241 (hereby expressly incorporated by reference in its entirety)) reveals the formation of a lipid salt. Thus, a need exists for improved methods of synthesis of amonafide salts. Additionally, improved pharmaceutically acceptable forms of amonafide and amonafide salts are desired.

### SUMMARY OF THE INVENTION

An object of this invention is to provide novel chemical synthesis of naphthalimides, for example, amonafide, in a scale suitable for pharmaceutical product development.

Another object of the present invention is to provide improved processes for preparing naphthalimide diammonium salts to improve pharmaceutical suitability.

In another aspect, this invention provides novel methods for the synthesis of naphthalimide analogs such as mitonafide, amonafide and their substituted salt forms.

In particular, one aspect of the invention includes a method of synthesis of a nitro naphthalimide comprising combining 3-nitro-1,8-naphthalic anhydride (NNA) in an organic solvent with an aliphatic diamine and refluxing. Another aspect of the invention includes a method of synthesis of mitonafide comprising adding 3-nitro-1,8-naphthalic anhydride in an organic solvent to N,N-dimethylethylenediamine (DMED) and refluxing.

One aspect of the invention includes a method of synthesis of an amonafide analog. The method comprises dissolving a mitonafide analog in an organic solvent and adding a reducing agent and a catalyst to the dissolved mitonafide analog to reduce the 3-nitro group of the mitonafide analog. A preferred aspect includes a method of synthesis of amonafide comprising dissolving mitonafide in an organic solvent and adding ammonium formate and a catalyst to the dissolved mitonafide.

A further aspect of the invention includes a method of synthesis of naphthalimide salts. According to this aspect, the method comprises dissolving a naphthalimide having at least two amines in an organic solvent such as tetrahydrofuran, and contacting the dissolved naphthalimide with an inorganic and/or organic acid to form the protonated form of the amines referred to herein as either a monoammonium or a diammonium salt. Organic acids include acetic acid, proprionic acid, glycolic acid, pyruvic acid, oxalic acid, maleic acid, malic acid, malonic acid, succinic acid, hydroxy succinic acid, fumaric acid, tartaric acid, citric acid, benzoic acid, cinnamic acid, mandelic acid, methanesulfonic acid, ethanesulfonic acid, p-toluenesulfonic acid and salicylic acid. Inorganic acids include hydrochloric acid, hydrobromic, acid, sulfuric acid, nitric acid and phosphoric acid.

In one embodiment, HCl gas or hydrochloric acid can be added to obtain amonafide dihydrochloride. The solution of amonafide dihydrochloride is cooled to form a precipitate, which is then filtered. The amonafide dihydrochloride precipitate may optionally be dissolved in water and precipitated using acetone for further purification.

In another embodiment, amonafide ammonium acetate could be formed by combining amonafide with excess acetic acid.

Also covered by the present invention are compositions. One embodiment includes a composition of naphthalimide as a diammonium salt wherein the salt requires two molar equivalents of alkali to produce a free base. Another composition includes a substituted 3-amino-naphthalimide as a salt. The substituted-3-amino-naphthalimide may be as a mono ammonium salt or a diammonium salt. According to one aspect, the diammonium salt is amonafide dihydrochloride. According to another aspect, the diammonium salt is amonafide dimesylate.

Also included in the invention are novel dosage forms of naphthalimides. One aspect includes an aqueous solution consisting essentially of amonafide. According to one aspect, the solution is substantially free of sugars. The solution is suitable for administration by injection, and comprises amonafide at between 1 and 250 mg/mL. According to another aspect, the solution comprises amonafide at between 10 and 100 mg/mL.

The pH of such an aqueous solution of a naphthalimide is between 4.0 and 7.0; alternatively, the pH may be between 5.5 and 6.5.

The solution is, according to some aspects, suitable for parenteral administration. The solution may be for intramuscular, subcutaneous, intravenous; intraperitoneal or intratumoral administration.

The solutions of the invention may also comprise a pharmaceutically acceptable carrier. The carrier may be provided at a concentration between about 0.1 to 100 mg/mL. The liquid dosage forms of the invention are injectable, sterile and/or stable. The solutions of the invention may be provided in a unit dosage form.

An additional aspect of the invention includes methods for manufacturing a sterile pharmaceutical composition comprising amonafide diammonium salts suitable for administration to a human. The method comprises solublizing an amonafide diammonium salt, such as ammonium dihydrochloride, in an aqueous solution; neutralizing the solubilized amonafide dihydrochloride with a molar equivalent of sodium hydroxide, adjusting the pH of the solubilized amonafide to about 6; and sterilizing the solubilized amonafide.

### DETAILED DESCRIPTION OF THE FIGURES

Figure 1 depicts the structure of the naphthalimide, amonafide.
Figure 2 depicts the structure of a 3-nitro-naphthalimide or mitonafide analog.
Figure 3 depicts the structure of a naphthalimide. The Q in the figure represents a substituent group, as described herein.
Figure 4 depicts chemical structures of several possible Q substituent groups that may substitute in the naphthalimide structure of Figure 3, or in the nitro-naphthalimide structure of

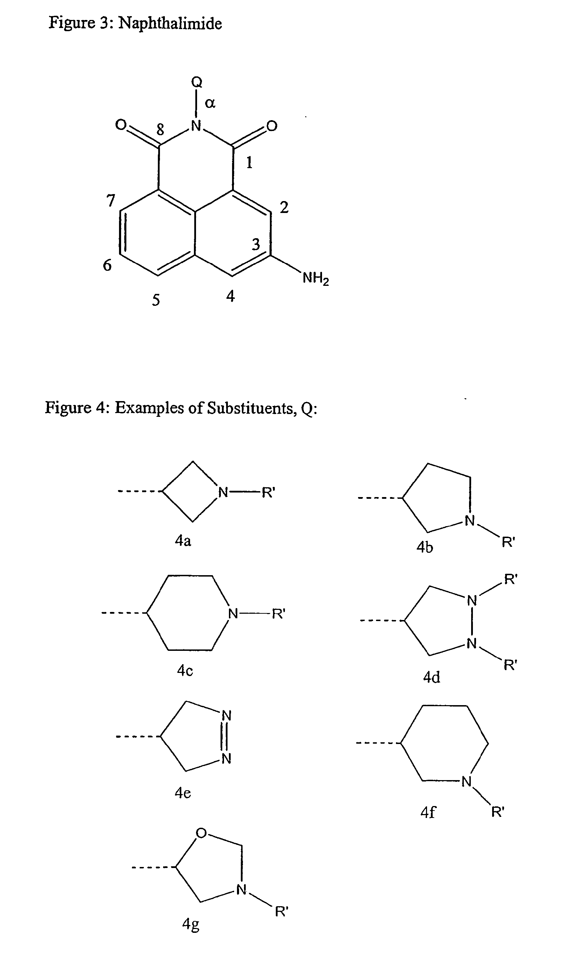

Figure 2. The ring structures each depict a bond to the amide nitrogen. This bond (marked by a dashed line) represents the point of attachment to the naphthalimide structure of Figure 3 or to the nitro-naphthalimide structure of Figure 2.

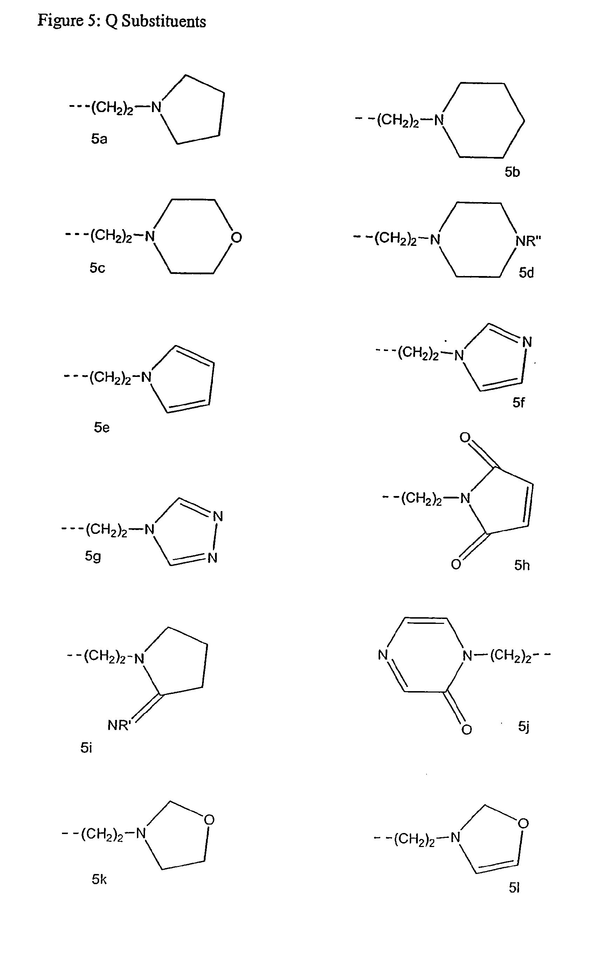

Figure 5 depicts another groups of possible Q substituent groups. Similar to those of Figure 4, these groups may substitute for Q in the naphthalimide structure of Figure 3, or in the nitro-naphthalimide structure of Figure 2. Each structure depicts a bond (maked by a dashed line), which represents the point of attachment of the substituent group to the naphthalimide structure of Figure 3 or to the nitro-naphthalimide structure of Figure 2.

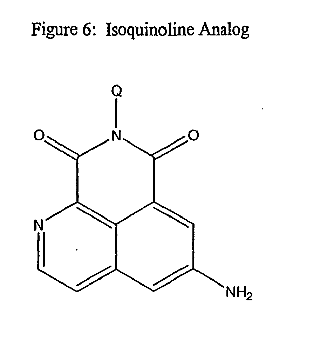

Figure 6 depicts the structure of an isoquinoline analog of amonafide. The Q in the figure represents a substituent group, as described herein.

### DETAILED DESCRIPTION OF THE INVENTION

### Methods of Synthesis

### i. Mitonafide Analogs

The invention includes methods of synthesis of a nitro naphthalimide, or a "mitonafide analog", as indicated in the structure in Figure 2. In one embodiment, the method comprises adding an aliphatic diamine to 3-nitro-1,8,-naphthalic anhydride in an organic solvent mixture, and refluxing to obtain the nitro naphthalimide. A general scheme depicting the reaction is below:

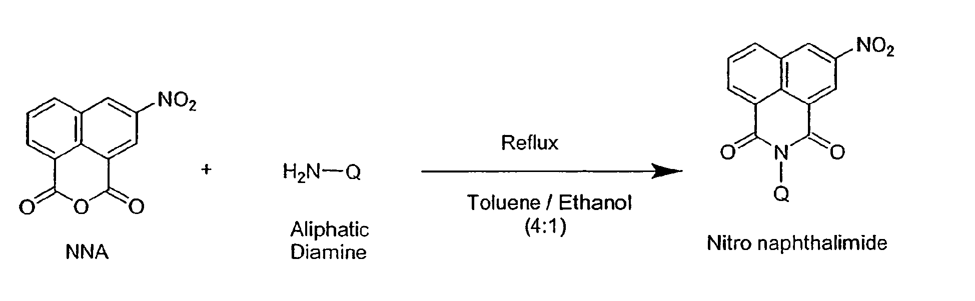

The aliphatic diamine used in the synthesis of a nitro naphthalimide can vary. The choice of aliphatic diamine allows synthesis of a mitonafide analog with, for example, a carbon chain length of 1-6. According to a preferred embodiment, the aliphatic diamine used is N,N-dimethylethylenediamine.

The structure in Figure 2 indicates a substituent group, Q. Q may represent a variety of structures, including those indicated in Figures 4 and 5.

One class of compounds synthesized by the methods of this invention includes naphthalimides with the structure depicted in Figure 3. The group Q in Figure 3 may be a variety of substituents, for example, the groups represented in Figure 4. For example, Q may be 1-R'-azetid-3-yl (Figure 4a), 1-R'-pyrrolid-3-yl (Figure 4b), 1-R'-piperid-4-yl (Figure 4c), 1,2-diR'-1,2-diazolid-4-yl (Figure 4d), 1,2-diazol-1-en-4y1 (Figure 4e), 1-R'-piperid-3-yl (Figure 4f), 3-R'-oxazolid-5-yl (Figure 4g). In Figure 4, R' = alkyl, unsaturated alkyl, acyl, alkoxy, aryl, amino, substituted amino, sulfo, sulfamoyl, carboxy, carbamyl, cyano.

In another embodiment, the structure in Figure 3 represents a naphthalimide of the invention, wherein Q represents -(CH₂)₂NR₂, wherein R= methyl, ethyl, propyl, butyl, etc. NR₂ in this representation may represent a heterocyclic group. Thus, Q may be any one of the groups shown in Figure 5.

In some embodiments, R₂ = -(CH₂)n- where n = 2 to 6, or R₂ = -(CH₂)mX-(CH₂)n- where m and n can be 0 to 5 and X can be NR" (where R" = hydrogen, alkyl, unsaturated alkyl, acyl, alkoxy, aryl, amino, substituted amino, sulfo, sulfamoyl, carboxy, carbamyl, cyano, or is not present), O, or S. Furthermore, these cyclic groups may have unsaturated bonds and may also bear substituents such as alkyl, aryl, or heteroaryl.

Further examples of the substituent group Q include, for example, those shown in Figure 5, which are 1-pyrrolidyl (5a), 3-R'-1-piperidyl (Figure 5b), morpholino (Figure 5c), 1-R'-piperazin-4-yl (Figure 5d), 1-pyrrolyl (Figure 5e), 1-imidazolyl (Figure 5f), 1,3,5-triazol-1-yl (Figure 5g), N-maleimido (Figure 5h), 2-(R'-imino)pyrrolidyl (Figure 5i), pyrazin-2-on-1-yl (Figure 5j), 3-oxazolidyl (Figure 5k), 3-oxazolyl (Figure 5l), and others known in the art, for example, 2-pyrrolyl, 3-chloro-1-pyrrolidyl, 2-nitro-1-imidazolyl, 4-methoxy-1-imidazolyl, 3-methyl-1-imidazolyl. In the structures depicted in Figure 5, R' = alkyl, unsaturated alkyl, acyl, alkoxy, aryl, amino, substituted amino, sulfo, sulfamoyl, carboxy, carbamyl, cyano, and other functional groups known to those skilled in the art.

Another group of compounds of the invention are naphthalimides having an amino group attached to other positions in the naphthalimide rings. According to one embodiment, the naphthalimide ring is modified to include one or more amino groups at positions other than position 3 of the naphthalimide ring. According to another embodiment, the naphthalimide ring is modified to include one or more amino groups at positions in addition to the amino group at position 3 of the naphthalimide ring. In another embodiment, the amino group at position 3 is replaced with a substituent group. Examples of such groups include: alkyl, aryl, nitro, substituted amino, sulfamoyl, halo, carboxy, carbamyl, cyano, and other functional groups known to those skilled in the art. In yet another embodiment, an additional group is attached to the naphthalimide ring also comprising an amino group at position 3. Examples of such substituent groups include: alkyl, aryl, nitro, amino, substituted amino, sulfamoyl, halo, carboxy, carbamyl, cyano, and other functional groups known to those skilled in the art.

Alternatively, the amino group at position 3 may be replaced by other substituent groups. Examples of substituent groups include: alkyl, aryl, nitro, substituted amino, sulfamoyl, halo, carboxy, carbamyl, cyano, and other functional groups known to those skilled in the art.

The naphthalene ring can be replaced with one bearing one or more nitrogen atoms in either or both rings. An example would be isoquinoline analogs (Figure 6), where Q is as previously defined. A preferred isoquinoline analog of amonafide is where Q is -(CH₂)n-N(CH₃)₂, where n is 1-12 or more. In a more preferred embodiment, n is 1-6. The isoquinoline analog may also have one or more substituent groups (as described herein for other analogs) reducing one or more hydrogens of the methyl and/or methylene groups.

An organic solvent is used in the method of the invention for refluxing the aliphatic diamine and 3-nitro-1,8,-naphthalic anhydride. In one embodiment, the organic solvent is ethanol. In another embodiment, the organic solvent is dimethylformamide. In yet another embodiment, the organic solvent is toluene-ethanol. In a preferred embodiment, the organic solvent is toluene-ethanol in a 4:1 ratio. (See Example 1).

The mixture is refluxed and monitored, for example, by thin-layer chromatography. Refluxing is performed according to one embodiment for 30 minutes. The resulting mixture is filtered and evaporated to obtain a brown solid of mitonafide or a mitonafide analog (see Figure 2).

Each of these naphthalimides may be converted into a mono or diammonium salt as discussed *infra.*

### ii. Naphthalimides

According to another embodiment, the invention includes a method of synthesis of a naphthalimide. In a preferred embodiment, the naphthalimide is amonafide (See Example 2). Amonafide is also known as 5-amino-2-[(dimethylamine)ethyl]-1H-benz[de-]isoquinoline-1,3-(2H)-dione.

The method of naphthalimide synthesis involves dissolving mitonafide or a mitonafide analog in an organic solvent. The organic solvent, according to one embodiment, is dichloromethane-methanol. In a preferred embodiment, dichloromethane-methanol is used in a ratio of 4:1 at 25 mL/g mitonafide.

The method of naphthalimide synthesis further involves adding a reducing agent (e.g., ammonium formate) to the dissolved mitonafide or mitonafide analog together with a catalyst. A variety of reducing agents suitable for reduction of the 3-nitro group are known in the art, including hydrazine, tetralin, ethanol, ascorbic acid, formic acid, formate salts, and phosphinic acid *(see, e.g.,* Johnstone, R. A. W. et al., Chemical Reviews 85 (2) 129 (1985); Entwhistle, I. D. et al., J. C. Soc. Perkin Trans. 1,443(1977)). According to a preferred embodiment, the reducing agent is ammonium formate. Other formate salts include substituted ammonium formates such as 2-hydroxyethylmethyl ammonium formate, methyl ammonium formate and morpholinium. According to a preferred embodiment, 4.5 mol equivalents of ammonium formate are used.

The method of naphthalimide synthesis involves use of a catalyst. A variety of suitable catalysts are known in the art, including the noble metals Pd, Pt, Rh and Raney Nickel (see, e.g., Johnstone, R. A. W. et al.(1985), *supra,* and Entwhistle, I. D. et al. (1977), *supra).* In one embodiment, the catalyst is palladium-carbon. In a preferred embodiment, 10% palladium-carbon (about 20% mitonafide weight) is added. The catalyst is mixed at room temperature under nitrogen for about 1 hour. The method further involves filtering the mixture and adding the mixture to a cool water bath (<10°C) to precipitate. After filtration, a precipitate forms which is dried to yield a naphthalimide, for example, amonafide (C₁₆H₁₇N₃O₂).

### iii. Naphthalimide salts

A further embodiment of the invention includes methods of synthesis of naphthalimide diammonium salts.

In general, naphthalimides are dibasic compounds containing at least two amines and in most cases an amine group covalently linked to an aromatic group. When in contact with an acid, at least one or two of the amines within the naphthalimide may be protonated by reaction with an inorganic or an organic acid to form salts. Such salts are generally weak acids comprising primary, secondary or tertiary ammonium ions formed by protonation of an amine within the amonifide molecule. The counter-ions for such ammonium ions can be any appropriate anion capable of being used in a pharmaceutical composition. In some embodiments, the acidic salts are formed by reacting the naphthalimide with a mineral (inorganic) or organic acid. Such mineral acids include hydrochloric acid, hydrobromic, acid, sulfuric acid, nitric acid and phosphoric acid. Some organic acids which may be used in forming salts of modified include acetic acid, proprionic acid, glycolic acid, pyruvic acid, oxalic acid, maleic acid, malic acid, malonic acid, succinic acid, hydroxy succinic acid, fumaric acid, tartaric acid, citric acid, benzoic acid, cinnamic acid, mandelic acid, methanesulfonic acid, ethanesulfonic acid, p-toluenesulfonic acid and salicylic acid. Inorganic acids include hydrochloric acid, hydrobromic, acid, sulfuric acid, nitric acid and phosphoric acid.

In most embodiments, two amines in the naphthalimide will be protonated ta form a diammonium salt. In preferred embodiments, at least 1.5, more preferably 1.75, still more preferably 1.9, still more preferably 1.95, still more preferably 1.99, and most preferably 2.0 mol equivalents of the two amines in the amonafide are protonated. In some instances where more than two amines are present, the possibility exists for protonation of all, or a portion of all, of the amines. For example, if three amines are present, least 1.5, more preferably 1.75, still more preferably 1.9, still more preferably 2.0, still more preferably 2.5, still more preferably 2.75, still more preferably 2.9, still more preferably 2.95, still more preferably 2.99, and most preferably 3.0 mol equivalents of the three amines are protonated.

In one embodiment, the amines of the naphthalimide have similar pK's for protonation. Upon titration of the free base amines of this embodiment with an acid, the amines are similarly protonated during the range of titration. In a preferred embodiment, at least two of the amines of the naphthalimide are greater than 50% protonated, more preferably greater than 75% protonated, still more preferably greater than 90% protonated, still more preferably greater than 95% protonated, still more preferably greater than 99% protonated, and most preferably 100% protonated.

In a further embodiment of the invention, the amines of the naphthalimide have different pK's for protonation. Upon titration of the amines with an acid, the amines will become protonated in a multiphasic manner according to their pK's. For example, the amine that has a higher pK value will become protonated before the amine that has a lower pK value when the free acid form of the naphthalimide is titrated with an acid. In a preferred embodiment, at least one of the amines of the naphthalimide is protonated and at least one of the amines of the naphthalimide is subsequently protonated, preferably greater than 50% protonated, more preferably greater than 75% protonated, still more preferably greater than 85% protonated, still more preferably greater than 95% protonated, still more preferably greater than 99% protonated and most preferably 100% protonated.

Diammonium salts of naphthalimide generally refers to naphthalimide salts which contain two protonated amines with the naphthalimide structure. Partial diammonium salts include those naphthalimides wherein at least 1.5 mol equivalents of the amines are protonated. In some embodiments, the counter-ions may be a mixture of one or more of the base forms of the aforementioned inorganic and/or organic acids.

In a preferred embodiment, the naphthalimide diammonium salt is amonafide dihydrochloride.

According to this embodiment, HCI gas is bubbled over amonafide solution to precipitate a salt form of amonafide. The process is robust and easy to scale up. Amonafide monohydrochloride as disclosed by US Patent No. 5,420,137 is manufactured by reaction with calculated amount of HCI solution. This process may result inaccurate amount of HCI in the final product and this process is not easy to scale up.

Dihydrochloride salt is more acidic and more soluble in water, as compared to a monohydrochloride salt. As a result, a wider range of drug concentration can be achieved to facilitate further manufacturing process such as lyophilization and more flexible to meet clinical needs.

A preferred embodiment of the present invention provides an improved synthesis of amonafide dihydrochloride salt exhibiting a well-defined crystalline structure with a narrow melting temperature range. The characteristic physical and chemical properties and stability of this form improve the safe handling of this cytotoxic drug during the manufacture of pharmaceutical dosage forms such as oral products including tablet and capsule forms, as well as a wide range of injectable dosage forms, such as liquid or lyophilized forms.

The creation of mono and diammonium salt forms enables the generation of pharmaceutically relevant dosages useful for the treatment of abenant cell conditions such as hyperproliferative diseases, including, for example, cancer and precancerous conditions.

### Pharmaceutical Dosage Forms and Modes of Administration

The National Cancer Institute has conducted clinical trials in cancer chemotherapy using a lyophilized amonafide product. Certain information regarding its chemistry and its pharmaceutical formulation are given in the publication titled AMONAFIDE (NSC-308847), NCI Investigational Drugs, Pharmaceutical data (1994). Notably, the dosage is a sterile 500 mg (as the base) vial. Constitution with 9.6 mL of Sterile Water for Injection, USP or 0.9% Sodium Chloride Injection, USP, results in a solution containing,50 mg/mL of amonafide with pH adjusted to 5 to 7 with sodium hydroxide. Reconstitution can be problematic if improperly performed and is better avoided.

One objective of the present invention, therefore, is to provide a stable, therapeutically acceptable, intravenously injectable dosage form of a naphthalimide or naphthalimide salt (e.g., amonafide) that does not require lyophilization and reconstitution, can be packaged and shipped as single vial instead of a dual-vial package, and can be supplied in a liquid formulation from 1-250 mg/mL. These objectives are achieved by the present invention, as described in detail below.

The compositions include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well known in the art of pharmacy.

Additives, carriers or excipients are well known in the art, and are used in a variety of formulations. See for example, Gilman, A.G. et al., eds., THE PHARMACOLOGICAL BASIS OF THERAPEUTICS, 8th Ed. Pergamon Press, New York, (1990), incorporated herein by reference in its entirety. In practical use, the compositions of the invention can be combined as the active ingredient in intimate admixture with a pharmaceutical carrier or excipient according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols (e.g., polyethylene glycol), oiis, alcohols, flavoring agents, sweeteners, preservatives, coloring agents and the like in the case of oral liquid preparations, such as, for example, suspensions, elixirs and solutions; or carriers such as starches (e.g. corn or other), sugars, lactose, serum albumin, microcrystalline cellulose, buffers (e.g., sodium acetate), diluents, granulating agents, lubricants, binders, disintegrating agents and the like in the case of oral solid preparations such as, for example, powders, hard and soft capsules and tablets, with the solid oral preparations being preferred over the liquid preparations.

### i. Oral Dosage Forms

Because of their ease of administration, tablets and capsules represent a particularly advantageous oral dosage unit form in which case solid pharmaceutical carriers are obviously employed. If desired, tablets may be coated by standard aqueous or nonaqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

### ii. Liquid Dosage Forms

The present invention also includes a liquid dosage form of a naphthalimide or naphthalimide salt prepared according to the methods described herein.

In one embodiment, the liquid dosage form prepared according to the methods of the invention is a stable sterile aqueous solution of a naphthalimide or naphthalimide salt (e.g., amonafide or amonafide dihydrochloride) in a sealed container such as an ampoule or vial, is in unit dosage form suitable for intravenous administration, has a concentration of a naphthalimide or naphthalimide salt between about 1 and about 250 mg/mL, and has a pH between about 3.0 and 7.0. In a preferred embodiment, the concentration of a naphthalimide or naphthalimide salt is about 20 mg/mL.

In a preferred embodiment, the pH of the liquid dosage form is about 6.0. Preferably, the pH is adjusted, if necessary, using a nontoxic, pharmaceutically and therapeutically acceptable inorganic source base. In a preferred embodiment, the base is a mineral base. In a more preferred embodiment, the base is sodium hydroxide.

In one embodiment, the liquid dosage form prepared according to the methods of the invention preferably is free of any other added chemicals. In another embodiment, the liquid dosage form contains a customary, physiologically acceptable excipient or carrier such as a preservative or tonicity agent. In one embodiment, an aqueous solution of amonafide comprises a carrier or excipient. Preferably, a carrier or excipient, when provided, is present at a concentration between about 0.1 mg/ml to 100 mg/ml.

According to one embodiment, the liquid dosage form is stable. "Stable" means that the liquid dosage form exhibits less than 5% loss of potency as measured by high performance liquid chromatography (HPLC) upon storage for 1 month at 60°C or 9 months 40°C.

The compositions of the invention may be conveniently presented in unit dosage forms, and prepared by any methods known in the art of pharmacy. The term "unit dosage form" refers to physically discrete units suitable as unitary dosages for human subjects and other mammals, each unit containing a predetermined quantity of active material calculated to produce the desired therapeutic effect in association with a suitable pharmaceutical excipient or carrier.

In the preferred embodiment, the pharmaceutical compositions are water soluble, such as being present as pharmaceutically acceptable salts, which is meant to include both acid and base addition salts. "Pharmaceutically acceptable acid addition salt" refers to those salts that retain the biological effectiveness of the free bases and that are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, maleic acid, malic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid and the like. "Pharmaceutically acceptable base addition salts" include those derived from inorganic bases such as sodium, potassium, lithium, ammonium, calcium, magnesium, iron, zinc, copper, manganese, aluminum salts and the like. Particularly preferred are the ammonium, potassium, sodium, calcium, and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, and ethanolamine.

The invention includes a method for manufacturing a sterile pharmaceutical composition. The pharmaceutical composition comprises an amonafide diacidic salt suitable for administration to a human. The method includes the steps:
(a) solubilizing amonafide diammonium salt in an aqueous solution;
(b) neutralizing the solubilized amonafide salt with a molar equivalent of base; e.g., sodium hydroxide;
(c) adjusting the pH of the solubilized amonafide salt to about 6; and
(d) sterilizing the solubilized amonafide dihydrochloride.

### RELEVANT LITERATURE

Dorr, R.T. and VonHoff, D.D. Cancer Chemotherapy Handbook 2nd Ed. 11994, Appleton and Lange.

Obregon, M.M., Gil, M.E., ruz, V., Bemabeu, J., Camacho, M.A., "Compatibility study of amonafide-HCI with hydrophobic excipients", Conference Proceedings, World Meet. Pharm., Biopharm, Pharm., Technol., 1^{st}.

Brana, M.F., Castellano, J.M., Moran, M., Emling, F., Kluge, M., Schlick, E., Klbe, G., Walker, N., "Synthesis, structure and antitumor activity of new benz[d,e]isoquinoline-1,3-diones", Arzneimittelforschung, 45 (12), 1311-8 (1995).

Camacho-Sanchez, M.A., Torres-Suarez, A.I., Sanz, M.P., "Stability of amonafide solutions in front of light and temperature", Clenc. Ind. Farm., 8(Mar-Apr), 104-109, (1989).

### EXAMPLES

The following examples, given without implied limitation, show how the invention can be put into practice.

### Example 1: Synthesis of Mitonafide

300 g 3-nitro-1,8-naphthalic anhydride (1.23 mol) and 129 g N,N-dimethylethylenediamine (1.46 mol) were added in toluene-ethanol mixture (4:1) and refluxed under nitrogen for 30 minutes. The process was monitored by thin-layer chromatography (TLC). After cooling, the reaction mixture was filtered and dried in a rotovap flask at about 50°C. 70% alcohol was added to the dried material and filtered. The collected material was dried under vacuum to yield a brown solid. A scheme for the reaction is below:

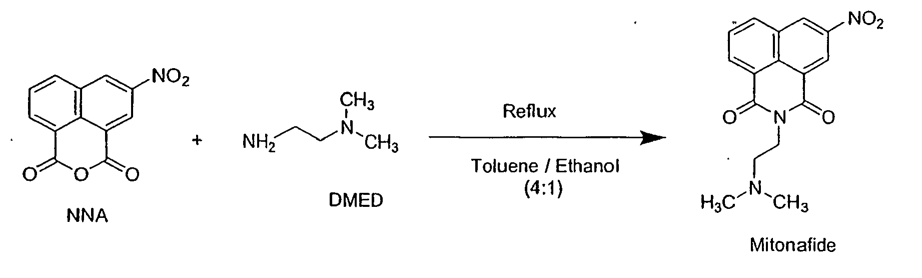

### Example 2: Synthesis of Amonafide

266 g crude mitonafide prepared as described in Example 1 was dissolved in dimethylformamide and methanol mixture (4:1) at 25 mL/g. Ammonium formate (4.5 mol eq) and 10% Paladium-carbon (about 20% mitonafide weight) were added. The reaction was monitored by TLC. The mixture was filtered, and the filtrate was precipitated in cool water. The precipitate was collected after filtration. After drying, 399 g of amonafide yellow solid were obtained. A scheme for the reaction is below:

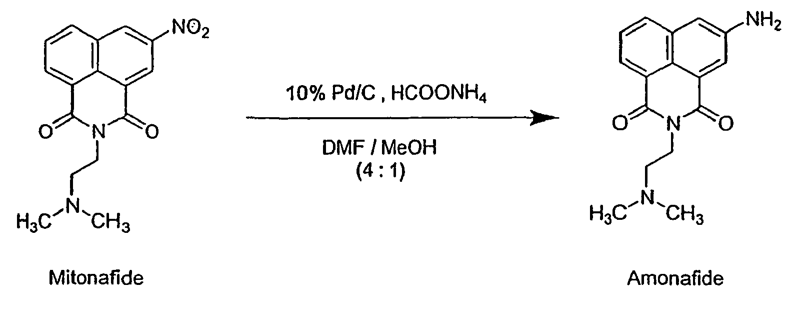

Amonafide produced have the purity greater than 99.6%, total related impurities less than 0.1% and the following characteristics: 1H-NMR, DMSO-d6, δ2.205 (S, 6H, Ha), 2.490 (t, 2H, Hb), 4.131 (t, 2H, Hc), 6.002 (s, 2H, disappeared after D₂O exchange, Hi), 7.283-8.085 (m, 5H, Hd, He, Hf, Hg, Hh); MS (MH = 284.2); elemental analysis: C₁₆H₁₇N₃O₂.

### Example 3: Synthesis of Amonafide Dihydrochloride

390 g amonafide was dissolved in THF (tetrahydrofuran) (100 mL/g) in an extraction flask and filtered through a 0.45-µm filter. The filtrate was cooled to below 10°C and bubbled with HCl gas until the pH reaches 1, to form precipitate. The precipitate was filtered and dried at 40°C under vacuum , resulting in 264 g of amonafide dihydrochloride 95% purity.

The precipitate may be optionally washed three times with each of tetrahydrofuran and diethyl ether. This material can be further purified by dissolving in water and reprecipitating, for example, by addition of acetone.

The synthetic process, as described above, produces crystalline powder, confirmed by x-ray diffraction, of amonafide dihydrochloride salt with melting point of 270°C. The structure and molcular weight as amonafide dihydrochloride was confirmed by elemental analysis (see Table 1), 1H-NMR and mass spectroscopy (not shown). The product exhibits an osmolality equivalent to 0.9% sodium chloride solution. Being isotonic enables one to inject this product by intramuscular, subcutaneous, intratumoral or intrathecal without local tissue side effects. Equal mols of NaOH are required to neutralize amonafide dihydrochloride during the formulation of the final dosage form, resulting in less manufacturing time for pH adjustment.

**Table 1. Elemental Analysis of Amonafide Dihydrochloride (Lot CB0717)**

| | %C | %H | %N | % C1 | %O |
|---|---|---|---|---|---|
| Theoretical | 53.94 | 5.38 | 11.80 | 19.90 | 8.98 |
| Found | 53.27 | 5.34 | 11.41 | 19.43 | 8.81 |

### Example 4: Solubility

Data from pH-solubility profiles indicate that amonafide dihydrochloride material is soluble in water with pH 1-2 and solubility greater than 250 mg/mL. Precipitation occurs as pH increases above 6.5. Solubility is about 1 mg/mL at pH 9 and 0.05 mg/mL at pH 11.

### Example 5: Titration with Sodium Hydroxide

561 mg amonafide dihydrochloride (1.57 mmol) was dissolved in 10 mL water to yield a clear red solution with pH at 1.2. With constant stirring, 1N NaOH solution was added. Solution remained clear until pH greater than 6.7. As shown in the titration curve below, 3.3 mmol NaOH was used to neutralize amonafide dihydrochloride in the solution. The calculated mol ratio of amonafide to NaOH was 1:2.

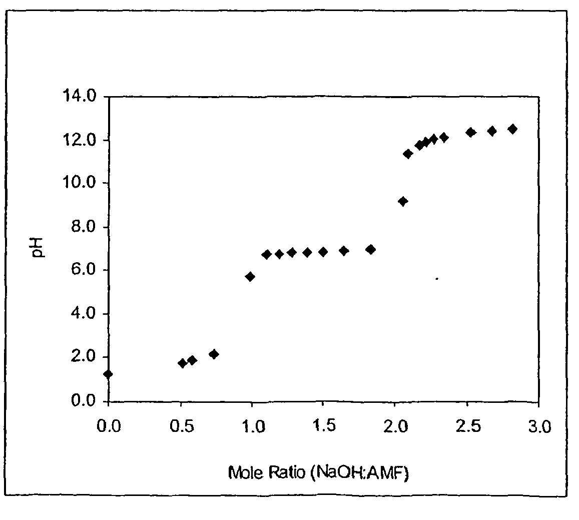

### Example 6: Preparation of Liquid Formulation

Amonafide 2HC1 was dissolved in 80% batch quantity of Water for Injection. Calculated sodium hydroxide, 2 mol equivalent, was slowly added to neutralize pH. Final pH was adjusted to 6.0 with diluted NaOH and HC1 solutions. Solution was then diluted to final volume with Water for Injection and mixed. The solution was sterilized by filtration and dispensed into 5-cc glass vials.

### Advantages of liquid product over lyophilized product

a. Lyophilization is an expensive manufacturing process (equipment, time, energy, etc.)
b. Lyophilized product requires dual vial pack containing lyophilized vial and diluent vial, extra manufacturing, packaging and labeling costs, extra room for storage, shipping.
c. More preparation steps for a lyophilized product, more hazardous waste generated, increased risk of contamination and safety.
d. Inaccurate dose due to improper reconstitution.

### Example 7: Administration of aqueous, stable, sterile amonafide

The liquid or lyophilized dosage forms can be administered by intravenous infusion by diluting the drug product in, for example, Sterile Water for Injection, Bacteriostatic Water for Injection, Dextrose (2.5%, 5%, 10%), Dextrose-saline combination, Fructose (10%), Fructose in saline, Ringer's Injection, Lactated Ringer's Injection, Sodium Chloride (0.45%, 0.9%) or combination with other chemotherapeutic drug.

Combinations of pharmaceutical compositions may be administered. Moreover, the compositions may be administered in combination with other therapeutics. Particular combinations of interest are described in U.S. Serial Nos. 09/834,177, 09/810,527, and 09/996,354, all of which are hereby incorporated by reference in their entirety.

### Example 8: Amonafide lyophilized product formulation

| | Per Vial |
|---|---|
| Amonafide 2HC1 | 125.75 mg |
| (amonafide | 100.0 mg) |
| Sodium Hydroxide | 29.0 mg |

### Example 9: Method of High-Performance Liquid Chromatography

Amonafide is chromatographed on a Keystone BDS Hypersil 5-µm C18 column. Detection is achieved by monitoring the UV absorbance at 344 nm and quantification is accomplished by peak area measurement with external calibration. This method is applicable to bulk powder and liquid dosage formulations. Specificity, linearity, precision and accuracy have been demonstrated.

The following clauses also form part of the disclosure of this application
1. A method of synthesis of a nitro naphthalimide comprising:
   combining 3-nitro-1,8-naphthalic anhydride and an aliphatic diamine in an organic solvent to form a solution; and
   refluxing said solution.
2. A method of synthesis of mitonafide comprising:
   combining 3-nitro-1,8-naphthalic anhydride and N,N-dimethylethylenediamine in an organic solvent to form a solution; and
   refluxing said solution.
3. A method of synthesis of an amonafide analog comprising combining a mitonafide analog comprising a 3-nitro group, ammonium formate, and a catalyst in an organic solvent to reduce said 3-nitro group.
4. A method of synthesis of amonafide comprising combining mitonafide, ammonium formate, and a catalyst in an organic solvent.
5. A method of synthesis of a naphthalimide diammonium salt comprising:
   dissolving a naphthalimide in an organic solvent; and
   contacting said dissolved naphthalimide with an inorganic or organic acid to form a naphthalimide diammonium salt.
6. The method of clause5 wherein said inorganic acid is selected from the group consisting of hydrochloric acid, hydrobromic, acid, sulfuric acid, nitric acid and phosphoric acid.
7. The method ofclause5 wherein said organic acid is selected from the group consisting of acetic acid, proprionic acid, glycolic acid, pyruvic acid, oxalic acid, maleic acid, malic acid, malonic acid, succinic acid, hydroxy succinic acid, fumaric acid, tartaric acid, citric acid, benzoic acid, cinnamic acid, mandelic acid, methanesulfonic acid, ethanesulfonic acid, p-toluenesulfonic acid and salicylic acid.
8. A composition comprising a naphthalimide as a diammonium salt.
9. A composition according toclause8 wherein the naphthalimide comprises amonafide as a diammonium salt formed by combining amonafide with an inorganic or organic acid.
10. A composition of clause 9 wherein said inorganic acid is selected from the group consisting of hydrochloric acid, hydrobromic, acid, sulfuric acid, nitric acid and phosphoric acid.
11. A composition of clause9 wherein said organic acid is selected from the group consisting of acetic acid, proprionic acid, glycolic acid, pyruvic acid, oxalic acid, maleic acid, malic acid, malonic acid, succinic acid, hydroxy succinic acid, fumaric acid, tartaric acid, citric acid, benzoic acid, cinnamic acid, mandelic acid, methanesulfonic acid, ethanesulfonic acid, p-toluenesulfonic acid and salicylic acid.
12. A composition according toclause8 wherein said diammonium salt is amonafide dimesylate.
13. A composition according to clause 8 wherein said diammonium salt is amonafide dihydrochloride.
14. A composition comprising a substituted 3-amino-naphthalimide salt.
15. An aqueous solution consisting essentially of a dissolved amonafide diammonium salt, said solution being suitable for administration by injection, said solution comprising amonafide at between 1 and 250 mg/mL, said solution having a pH between 4.0 and 7.0.
16. An aqueous solution of amonafide according to clause 15 suitable for parenteral, intramuscular, subcutaneous, intravenous, intraperitoneal or intratumoral administration.
17. An aqueous solution of amonafide, said solution being suitable for administration by injection, said solution comprising amonafide at between 10 and 100 mg/mL, and said solution having a pH between 5.5 and 6.5.
18. The solution according to clause 17, wherein said solution is substantially free of sugars.
19. The solution according to clause17, wherein said solution further comprises a pharmaceutically acceptable carrier.
20. The solution according to clause19, wherein said carrier is provided at a concentration between about 0.1 to 100 mg/mL.
21. The solution according to clause 17, wherein said solution is provided in a unit dosage form.
22. A method for manufacturing a sterile pharmaceutical composition comprising a naphthalimide diammonium salt suitable for administration to a human, said method comprising:
   (a) solubilizing a naphthalimide diammonium salt in an aqueous solution;
   (b) neutralizing the aqueous solution with a molar equivalent of base;
   (c) adjusting the pH of the solution comprising said solubilized naphthalimide diammonium salt to about 6; and
   (d) sterilizing said solution.

## Claims

1. A method of synthesis of an amonafide salt comprising: dissolving amonafide in an organic solvent; and contacting said dissolved amonafide with an inorganic acid selected from the group consisting of hydrochloric acid, hydrobromic acid, sulphuric acid, nitric acid and phosphoric acid or an organic acid selected from the group consisting of acetic acid, propionic acid, glycolic acid, pyruvic acid, maleic acid, malic acid, malonic acid, succinic acid, fumaric acid, tartaric acid, citric acid, mandelic acid, salicylic acid, methanesulfonic acid, oxalic acid, hydroxy succinic acid, benzoic acid, cinnamic acid, ethanesulfonic acid and p-toluenesulfonic acid to form an amonafide salt.

2. A method according to claim 1, wherein the inorganic acid is selected from the group consisting of hydrobromic acid, sulphuric acid, nitric acid and phosphoric acid and the organic acid is selected from the group consisting of methanesulfonic acid, oxalic acid, hydroxy succinic acid, benzoic acid, cinnamic acid, ethanesulfonic acid and p-toluenesulfonic acid to form an amonafide salt.

3. A composition comprising an amonafide salt wherein said salt is formed by combining amonafide with an inorganic acid selected from the group consisting of hydrochloric acid, hydrobromic acid, sulphuric acid, nitric acid and phosphoric acid or an organic acid selected from the group consisting of acetic acid, propionic acid, glycolic acid, pyruvic acid, maleic acid, malic acid, malonic acid, succinic acid, fumaric acid, tartaric acid, citric acid, mandelic acid, salicylic acid, methanesulfonic acid, oxalic acid, hydroxy succinic acid, benzoic acid, cinnamic acid, ethanesulfonic acid and p-toluenesulfonic acid.

4. A composition according to claim 3, wherein the inorganic acid is selected from the group consisting of hydrobromic acid, sulphuric acid, nitric acid and phosphoric acid and the organic acid is selected from the group consisting of methanesulfonic acid, oxalic acid, hydroxy succinic acid, benzoic acid, cinnamic acid, ethanesulfonic acid and p-toluenesulfonic acid to form an amonafide salt.

5. A composition according to claim 4 wherein said salt is amonafide dimesylate.

6. An aqueous solution consisting essentially of water and the composition of claim 4, said solution being suitable for administration by injection, said solution comprising amonafide at between 1 and 250 mg/mL, said solution having a pH between 4.0 and 7.0.

7. An aqueous solution of amonafide according to claim 6 suitable for parenteral, intramuscular, subcutaneous, intravenous, intraperitoneal or intratumoral administration.

8. The aqueous solution of claim 6, wherein said solution is suitable for administration by injection, said solution comprising amonafide at between 10 and 100 mg/mL, and said solution having a pH between 5.5 and 6.5.

9. The solution according to claim 8, wherein said solution is substantially free of sugars.

10. The solution according to claim 8, wherein said solution further comprises a pharmaceutically acceptable carrier.

11. The solution according to claim 10, wherein said carrier is provided at a concentration between about 0.1 to 100 mg/mL.

12. The solution according to claim 8, wherein said solution is provided in a unit dosage form.

13. A method for manufacturing a sterile pharmaceutical composition comprising an amonafide salt suitable for administration to a human, said method comprising:
(a) solubilizing the composition of claim 4 in an aqueous solution;
(b) adjusting the pH of the solution comprising said composition to about 6; and
(c) sterilizing said solution.
